# EUROPEAN PATENT SPECIFICATION

(11) **EP 0 184 701 B1**
(45) Date of publication and mention of the grant of the patent: **08.06.1994**
(21) Application number: 85114835.3
(22) Date of filing: 22.11.1985
(51) Int. Cl.: G01N 33/543, G01N 33/532, G01N 33/76

(54) **A method for determining a ligand**
Verfahren zur Bestimmung eines Liganden
Procédé pour déterminer un ligand

(30) Priority: 03.12.1984 US 677429; 31.05.1985 US 739784
(43) Date of publication of application: 18.06.1986
(73) Proprietor: HOECHST CELANESE CORPORATION, Somerville, N.J. 08876 (US)
(72) Inventor: Wong, George, San Diego, CA 92116 (US)
(74) Representative: Beck, Bernhard

(56) References cited:
- EP-A- 0 124 366
- EP-A- 0 132 292
- DE-A- 2 915 082
- US-A- 4 149 003
- US-A- 4 228 237
- US-A- 4 550 075
- BIOCHEMICAL JOURNAL, vol. 173, 1978, Cambridge (GB); J. CARLSSON et al., pp. 723-737#

## Description

### 1. Field of the Invention

This invention relates to a method for determining a ligand and, more particularly, to a method utilizing a specific binding substance for the ligand labeled with an avidin reagent covalently bonded thereto and a biotin labeled enzyme reagent.

### 2. Discussion of the Prior Art

U.S. Patent 4,228,237 is directed to a method of determining a ligand utilizing enzyme labeled avidin reagent and biotin labeled specific binding substance for the ligand, the latter being used to provide a biotin bridge to couple the specific binding substance to the enzyme labeled avidin.

It is advantageous in many instances to employ complexes and conjugates of higher specific enzyme activity and accordingly a method to achieve this result is desired and needed.

### SUMMARY OF THE INVENTION

This invention relates to a method for determining a ligand, and more particularly, to a method utilizing a specific binding substance for the ligand labeled with an avidin reagent covalently bonded thereto and a biotin labeled enzyme reagent.

The method comprises providing an insoluble phase containing a specific binding substance for the ligand. The insoluble phase is incubated with the following reagents:
(i) a medium suspected of containing the ligand;
(ii) a soluble reagent selected from the group consisting of (a') a specific binding substance for the ligand which is covalently linked with avidin;
   (b') the reagent (a') complexed with biotin labeled enzyme or (c') ligand which is linked to avidin or (d') reagent (c') complexed with biotin labeled enzyme; and
(iii) soluble biotin labeled enzyme when the reagent is (a') or (c'). The unreacted reagents are separated from the insoluble phase after incubation and the enzyme activity of either the separated unreacted reagents or the separated insoluble phase is then measured to determine the ligand.

### DETAILED DESCRIPTION

Although the biotin-avidin system of the present invention may be utilized in any conventional heterogeneous binding process, non-competitive and competitive binding processes are preferred.

Generally, the components of the specific binding reaction (the insoluble phase containing a specific binding substance for the ligand and the appropriate reagents) may be added together in a manner or sequence which provides that the resultant enzyme activity is easily measurable in a subsequent detection reaction system.

Ligands which may be determined according to the instant invention are specific organic materials for which a specific binding substance can be provided. A specific binding substance is any substance or group of substances having a specific binding affinity for the ligand to the exclusion of other substances. When a non-competitive process is employed, the specific binding substance contained in the insoluble phase and the specific binding substance bound to an avidin reagent, destined to be employed in the process of the invention, may be identical or they may differ.

Generic classes of materials embraced by the term ligand include, for example, antigens, antibodies and haptens as well as those substances having naturally occurring receptors or for which receptors can be made, e.g. DNA probes.

When the ligand is an antigen, specific binding substance utilized to detect the antigen is normally the corresponding antibody produced when the antigen is introduced into the blood stream of a vertebrate. Examples of antigens which may be determined according to the instant invention include, for example, polypeptide and protein hormones, human IgE and alpha₁ fetoprotein, a fetal antigen that also occurs in serum of patients with hepatoma and embryonal adrenocarcinoma. Conversely, when the ligand is an antibody the eliciting antigen may be employed as a specific binding substance. Assay of antibody titers is particularly useful in the diagnosis of, for example, infectious diseases such as syphilus, rubella and infection caused by haemolytic streptococci.

When the ligand is a hapten (i.e., a protein-free substance which does not itself elicit antibody formation), specific binding substance utilized to detect the hapten is an antibody produced when the hapten, bound to an antigenic carrier, is introduced into the blood stream of a vertebrate. Examples of haptens which may be determined according to the instant invention include steroids such as estrone, estradiol, testosterone, pregnanediol and progesterone; vitamins such as B₁₂ and folic acid; triodothyronine, thyroxine, histamine, serotonine, digoxin, prostaglandins, adrenalin, noradrenalin, morphine, vegetable hormones and antibiotics such as penicillin.

When the ligand is a substance having a naturally occurring receptor, or a receptor which is made, the receptor can be utilized as the specific binding substance for detecting the ligand, provided the receptor can be isolated in a form specific for the ligand. Illustrative ligands which have naturally occurring receptors include thyroxine, many steriods, polypeptides and neuropolypeptides such as insulin, gastrin, angiotensin, endorphins, various opiates, and many others. Receptors for this class of ligands are usually proteins.

A liquid sample medium, suspected of containing the ligand to be determined is selected. The liquid medium which is presumed to contain the ligand to be determined may be a natural or synthetic liquid. In many instances, the liquid will be a biological fluid. Biological fluids which are examinable for ligands according to the instant invention include, for example, whole blood, serum, plasma, urine, amniotic fluid and cerebrospinal fluid.

A suitable insoluble phase containing a specific binding substance to which the ligand in the sample is destined to be exposed to and to which it is to be bound, is prepared. A suitable insoluble phase includes any conventional insoluble support or carrier which is employed for biochemical analyses. Some typical solid carriers or support substances used in the instant invention may include insoluble organic or partly inorganic polymers such as cellulose, nitrocellulose, agarose, polypropylene, polystyrene, cross-linked dextran, nylon and glass derivatives. Common configurations for such supports include beads, balls, paper discs, tubes, microtiter plates, films, mixing sticks and slides.

Preparation of the insoluble phase containing specific binding substance for the ligand to be determined can be accomplished by known methods. For example, the specific binding substance can be attached to a solid carrier by cross-linking, by covalent bonding or by physical adsorption. For example, when the ligand to be detected is an antigen, preparation of the insoluble phase can be accomplished by simply coating the tubes or plates with the appropriate antibody. When nylon beads are used, the appropriate antibody may be covalently coupled to the beads by the method of Faulstich et al described in FEBS Letters, 48, 226, (1974).

Destined to be reacted with the ligand bound to the insoluble phase is a specific binding substance for the ligand, covalently bonded with avidin reagent, and an enzyme reagent which is itself biotin labeled. The avidin reagent which is covalently bound to the specific binding substance includes avidin obtained from egg whites or strepavidin from streptomyces avidinii.

The avidin reagent may be covalently bound to the specific binding substance or ligand by addition of external bivalent and polyvalent coupling reagents or in some cases by direct condensation of existing functional groups under standard conditions well known in the art. Such coupling reagents include gluteraldehyde, carbodiimides, diisocyanates and heterofunctional crosslinkers such as N-gamma-maleimidobutyryloxysuccinimide (GMBS) to form non-splittable bonds or thiolinkers such as N-succinimidyl-3-(2-pyridylthio)-propionate (SPDP) which can be used to form bonds of the splittable type.

Other coupling reagents are of the formula R¹-S-S-A-Z where R¹ is 2-pyridyl, 5-nitro-2-pyridyl or 4-pyridyl, A is a hydrocarbon residue having 1-10̸ carbon atoms, preferably 1-6 carbon atoms, and Z is a group 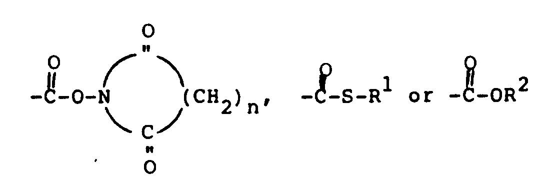
or acid addition salts of the last mentioned group, where n is 2 or 3, R¹ has the same significance as R¹ above and is equal thereto, and R² is methyl or ethyl. The hydrocarbon residue A is preferably an aliphatic residue but may also be an aromatic residue. Such coupling reagents and their preparation are revealed in U. S. Patent 4,149,0̸0̸3.

The covalent bonding of the specific binding substance and the avidin reagent by means of the splittable bridge may be accomplished in the manner revealed in U.S. Patent 4,231,999.

Although a wide variety of enzymes can be used to prepare the biotin labeled enzyme reagent, certain enzymes are preferred. For example, in qualitative determination of a ligand, the enzyme should preferably be detected by a color reaction.

Enzymes suitable for use in the instant invention include those classified as oxidoreductases, hydrolases and lyases according to the International Union of Biochemists (I.U.B.). Of the oxidoreductases, those acting on the CHOH group, the aldehyde or keto group or the CHNH₂ group of donors (1.1, 1.2 and 1.4 respectively) and those acting on hydrogen peroxide as acceptor (1.11) are preferred. Particularly preferred oxidoreductases include, for example, glucose oxidase and horseradish peroxidase. Of the hydrolases, of particular interest are those acting on ester bonds (both organic and inorganic esters) and those acting on glycosyl compounds, particularly glycoside hydrolases, namely 3.1 and 3.2 respectively. Particularly preferred hydrolases include, for example, alkaline phosphatase and φ-galactosidase.

Preparation of the soluble biotin labeled enzyme reagent may be typically accomplished through the use of biotin N-hydroxysuccinimide ester (BHNS) which reacts with free amino groups to form an amide linkage, or with biotin hydrazide which reacts with aldehyde groups from periodate-oxidized glycoproteins to form hydrazones.

A particularly preferred non-competitive binding process is the "Sandwich" technique wherein the components of the binding reaction comprise an insoluble phase containing a specific binding substance for the ligand and the following reagents:
(i) a measured amount of liquid medium suspected of containing the ligand;
(ii) avidin labeled specific binding substance for the ligand; and
(iii) soluble biotin labeled enzyme.
In an alternative mode, a preformed complex of avidin labeled specific binding substance and biotin labeled enzyme may be used in lieu of reagents (ii) and (iii). Such a reagent may be prepared by simply mixing reagents (ii) and (iii) together in appropriate quantities prior to incubation with ligand bound to the insoluble phase.

In the non-competitive binding process, the insoluble phase, under certain conditions may be incubated with the reagents in the presence of one another, or alternatively the insoluble phase may be incubated with the reagents individually in a precise sequence. The latter method is generally preferred.

When the insoluble phase is incubated with individual reagents in a precise sequence, the binding process can be effected in multiple, e.g. two or three, incubation periods.

In a three step incubation process, the insoluble phase is first reacted with the ligand reagent, i.e., a measured amount of liquid medium suspected of containing the ligand. Unreacted reagent is then removed and avidin labeled specific binding substance for the ligand is added. Following the reaction of the avidin labeled specific binding substance, unreacted avidin labeled reagent is removed and biotin labeled enzyme is added. Following the reaction of biotin labeled enzyme, unreacted reagent is separated from the insoluble phase and the enzyme activity of either the insoluble phase or the separated unreacted biotin labeled enzyme is determined by a suitable detection reaction. The enzymatic activity of the insoluble phase is directly related to the amount of ligand in the liquid medium whereas the enzymatic activity of separated unreacted biotin labeled enzyme is inversely related to the amount of ligand.

When avidin labeled specific binding substance is prereacted with biotin labeled enzyme, the binding process can be affected in two steps by substituting the avidin labeled reagent bound to biotin labeled enzyme for the individual avidin labeled and biotin labeled enzyme reagents.

In order to obtain quantitative results in the foregoing non-competitive binding process, the quantity of insoluble phase mixed with the reagents is generally larger than that quantity which is capable of forming bonds with the total amount of ligand assumed to be in the liquid medium. The quantity, in practice, is chosen in conformity with the aforementioned criteria based on the assumption that the ligand is present in the liquid at the highest concentration possible. The non-competitive process differs from conventional radioimmunoassay (RIA) in that in the first instance, the ligand to be determined is assayed directly by reaction with excess labeled binding substance whereas in the second instance, the ligand to be determined is in competition with labeled ligand for a limited amount of binding substance. Non-competitive binding is particularly suited to the detection of high molecular weight ligands with multiple binding sites.

If a competitive binding process is used, the components of the binding reaction comprises an insoluble phase containing a specific binding substance for the ligand and the following reagents.
(i) (A) a measured amount of liquid medium suspected of containing the ligand and (B) a known quantity of avidin labeled ligand and
(ii) soluble biotin labeled enzyme.
In an alternative mode, (B) may be avidin labeled ligand bound to biotin labeled enzyme thereby eliminating the need for reagent (ii). This complex reagent is prepared as previously described.

In a competitive binding assay employing reagents (i) and (ii), the insoluble phase may be incubated with the reagents in the presence of one another, or alternatively the insoluble phase may be incubated with the reagents individually in a particular sequence. In the latter mode, the insoluble phase is first incubated with a reagent comprising (A) a measured amount of liquid medium suspected of containing the ligand and (B) a known quantity of avidin labeled ligand. A competition is established between the ligand to be determined and avidin labeled ligand for the binding sites of the insolubilized specific binding substance. After equilibration, unreacted reagent is removed and biotin labeled enzyme reagent is added. Following the reaction of the biotin labeled enzyme reagent, unreacted reagent is separated from the insoluble phase and the enzyme activity of either the insoluble phase or the separated unreacted biotin labeled enzyme is determined by a suitable detection reaction. The enzyme activity of the insoluble phase is inversely related to the amount of ligand in the liquid medium, whereas the enzyme activity of the separated unreacted biotin labeled enzyme is directly related to the amount of ligand.

When avidin labeled ligand is pre-reacted with biotin labeled enzyme, the binding process can be effected in one incubation period by simply incubating the insoluble phase with a reagent comprising (A) a measured amount of liquid medium suspected of containing the ligand and (B) a known quantity of avidin labeled ligand bound to biotin labeled enzyme.

In order to obtain quantitative results in the competitive binding process, the quantity of insoluble phase specific binding substance per sample is usually less than that amount which is capable of binding the total quantity of ligand presumed to be present in the liquid and less than the total quantity of avidin labeled ligand or avidin labeled ligand bound to biotin labeled enzyme. In practical applications, this quantity is selected to correspond to the aforementioned criteria, based on the assumption that the largest quantity of ligand which can be present in the liquid medium is indeed there. In general, the quantity of avidin labeled ligand or avidin labeled ligand bound to biotin labeled enzyme which is brought in contact with the liquid medium does not exceed the smallest quantity of ligand to be determined in the liquid medium. The competitive binding method is especially suitable for detecting ligands which have few binding sites.

Ligands which are determined according to the instant invention with the aid of a non-competitive binding process (i.e., the "Sandwich" technique) must have at least two binding sites in order to bind with both the insoluble phase containing specific binding substance and avidin labeled specific binding substance. The foregoing criteria is not necessary when a competitive binding process is employed.

The determination of enzyme activity in the instant invention is accomplished with a detection reaction system suitable for the particular enzyme employed. Since there are differences, not only between assays for different enzymes, but even in the variety of assays for a particular enzyme, no general description of the assay techniques can be given; however, a wide diversity of media, conditions and substrates suitable for enzymes employed herein can be found in Bergmeyer. Methods of Enzymatic Analysis, Academic Press, New York, 1965.

Since the ratio of avidin covalently linked with specific binding substances need not be limited to a ratio of 1:1 and each avidin is capable of binding four molecules of biotin, it is now possible to obtain a greater enzyme to specific binding substance ratio than was heretofore realized by the use of the biotin labeled enzyme-avidin labeled specific binding substances. Accordingly, the resultant assay for ligand is more sensitive and accurate due to the resultant complexes or conjugates having higher enzyme specific activity.

### DESCRIPTION OF SPECIFIC EMBODIMENTS

The following specific descriptions is given to enable those skilled in the art to more clearly understand and practice the present invention. It should not be considered as a limitation upon the scope of the invention but merely as being illustrative and representative thereof.

### Example I

A procedure for the determination of human antibodies (IgG) to double stranded DNA using the "Sandwich" technique was carried out.

### (a) Preparation of Affinity Purified Goat Antibody (IgG) to Human Antibody (IgG) Covalently Linked to Avidin.

Purified goat antibody (IgG) to human antibody (IgG) covalently linked to avidin by means of N-succinimidyl-3-(2-pyridylthio) propionate (SPDP) was prepared in the following manner.

Combined were 3.7 mg egg white avidin dissolved in 1.0̸ mL of 0̸.0̸5 M phosphate buffer (pH 7.4) and 10̸0̸ ml of 0̸.256 M SPDP in ethanol. The resultant mixture was stirred 1 hour at 20̸^{o}C and the unreacted SPDP was removed therefrom by gel filtration on Sephadex® G-25 column.

Combined was 2.1 mg Goat anti-human IgG (affinity purified) dissolved in 1.0̸ mL of 0̸.0̸5 M phosphate buffer (pH 7.4) and 10̸0̸ ml of 0̸.256 M SPDP in ethanol. The resultant mixture was stirred for 1 hour at 20̸^{o}C and unreacted SPDP was removed therefrom by gel filtration on Sephadex® G-10̸0̸ column.

125 mL of 0̸.1 M sodium acetate (pH 4.5) was added to 1.25 mg SPDP- Goat anti-human IgG in 40̸0̸ mL volume (pH 5.0̸). To this was added 50̸ mL of 0̸.1 M dithiothreitol (DTT). The resultant mixture was stirred and then incubated for 15 minutes at 20̸^{o}C. The unreacted DTT was removed by gel filtered on Sephadex® G-25 column.

To 0̸.73 mg of SPDP Goat anti-human IgG (reduced) was added 1.24 mg SPDP Avidin. The resultant mixture was stirred gently for 1.5 hours at 20̸^{o}C. The resultant conjugate was then stored at 4^{o}C.

### (b) Determination of Human Antibodies (IgG) to Double Stranded DNA

To human double stranded DNA coated microtiter polystyrene wells (250̸ ul capacity) was added 10̸0̸ uL of human serum sample containing antibody (IgG) to double stranded DNA. The wells and sample were then incubated at 20̸^{o}C for 2 to 4 hours and then washed twice with wash buffer. To each well 10̸0̸ uL of diluted goat antibody (IgG) to human IgG-avidin conjugate reagent of (a) above was added and each well incubated for one hour at 20̸^{o}C. Excess reagent was removed by suction, and each well washed twice with wash buffer. To each well was then added 10̸0̸ uL biotin labeled alkaline phosphatase reagent which is commercially available. Each well was then incubated with the biotin labeled alkaline phosphatase reagent for 30̸ minutes at 20̸^{o}C whereafter excess biotin reagent was removed by suction and each well washed twice with wash buffer.

To each well was added 10̸0̸ uL p-nitrophenyl phosphate substrate reagent and incubated at 20̸^{o}C for 30̸ minutes, whereafter 10̸0̸ uL of 2N H₂SO₄ was added thereto. The optical density (O.D.) at 410̸ nm was read to obtain the enzyme concentrations.

### Example II

A procedure for the determination of human chorionic gonadotropin (hCG) can be carried out in the following manner.

### a) Preparation of Mouse Monoclonal Antibody (IgG) to hCG, α-Subunit Specific, Fab' Fragments Covalently Linked to Streptavidin

10̸.0̸ mg (0̸.166 umol) streptavidin is dissolved in 1.0̸ mL 0̸.0̸5 M phosphate buffered saline, pH 7.2. To this add 33 uL of a 5 mM solution of N-gamma-maleimidobutyryloxysuccinimide (GMBS) in tetrahydrofuran (THF) in dropwise fashion and stir gently for 1.5 hours at 20̸^{o}C.

Dissolve 50̸ mg purified mouse monoclonal antibody to α-hCG in 2.5 mL 0̸.1 M sodium acetate buffer (pH 4.5). To this, add 10̸0̸ uL of a 10̸ mg/mL solution of pepsin in acetate buffer, mix gently and allow to digest overnight (about 16 hours) at 37^{o}C. Neutralize mixture with 30̸0̸ uL 2M tromethamine (Tris). Apply digest to a G-20̸0̸ column (1.5 x 30̸ cm) and elute with 0̸.0̸1 Tris buffered saline (pH 7.3). Collect 2.5 mL fractions and monitor at 280̸ nm. The first major peak is F(ab')₂. Pool peak fractions and concentrate to 5 mL,(approximately 6mg/mL). To this add 0̸.5 mL 1 M Tris-HCl (pH 7.5), 50̸ uL 0̸.2 M ethylenedinitrilotetraacetic acid (EDTA) disodium salt and 50̸ uL 0.1 M dithiothreitol in 1 M Tris-HCl (pH 7.5). Gently mix and incubate at 20̸^{o} for 1 hour.

Apply mixture to a G-20̸0̸ column and elute as per peptic digest. Pool the first major peak fractions containing Fab' fragments. Immediately follow by mixing pooled Fab' mouse anti α-hCG solution with the GMBS treated stretavidin and gently stir for 3 hours at 20̸^{o}C. Quench excess maleimide with 50̸ uL 10̸ mM mercaptoethanol. Separate the conjugate from unreacted components by gel filtration through a Sephadex® G-20̸0̸ column. Elute with PBS and monitor at 280̸ nm. The first major peak contains the mouse anti α-hCG Fab'-streptavidin conjugate.

### b) Determination of Human Chorionic Gonadotropin (hCG)

To polystyrene microtiter wells coated with affinity purified mouse monoclonal antibody (IgG) to hCG, φ-subunit specific, add 10̸0̸ uL of patient serum sample, or hCG standard. Incubate wells for 3 hours at 20̸^{o}C followed by the aspiration-wash procedure described in Example I. To each well add 10̸0̸ uL of conjugate at working concentration and incubate for 1 hour at 20̸^{o}C. Follow aspiration and wash with addition of 10̸0̸ uL biotin-alkaline phosphate conjugate. Follow incubation and enzyme substrate reaction described in Example I. Use standard curve to determine sample hCG values.

### Example III

A procedure to detect autoantibodies to pancreatic islet cells can be carried out in the following manner.

### a) Preparation of Affinity Purified Goat Antibody (IgG) to Human Antibody (IgG) Covalently Linked to Avidin

Avidin from Streptomyces avidinii is conjugated to affinity purified goat antibody to human IgG according to the following procedure.

3.4 mg (0̸.0̸23 »mol) affinity purified goat antibody to human IgG is dissolved in 0̸.5 mL 0̸.0̸1 M phosphosaline buffer (PBS). While stirring add 3.0̸ uL (0̸.30̸ umol) N-succinimidyl-S-acetylthioacetate (SATA) in N,N-dimethylformamide (DMF) and incubate reaction mixture for 20̸ minutes at 20̸^{o}C. Chromatograph reaction mixture on a 15mL Sephadex® G-25 column with 0̸.0̸5M sodium phosphate buffer (pH 6.5) with 1 mM EDTA. Adjust the pH of the fraction containing 0̸.9 mg goat anti-human IgG-SATA to pH 7.5 and add 10̸0̸ uL 0̸.0̸5 M sodium phosphate buffer (pH 7.5) with 0̸.4 M NH₂OH·HCl and 0̸.0̸2 M EDTA. Stir mixture for 1.5 hours at room temperature and store at 4^{o}C.

Dissolve 1 mg (0̸.0̸16 umol) streptavidin in 1.0̸ mL 0̸.0̸5 M sodium phosphate buffer (pH 7.5) and add 27 uL (0̸.0̸65 umol) of 2.4 mM N-succinimidyl-3-(2-pyridylthio)propionate (SPDP) in dimethylsulfoxide. Stir reaction mixture for 1 hour at 20̸^{o}C and chromatograph on a 15mL Sephadex® G-25 column with 0̸.0̸5 M monobasic sodium phosphate (pH 4.3).

To 0̸.4 mg of goat anti-human IgG-SATA (0̸.8 mg/mL, add 0̸.48 mg streptavidin-SPDP (0̸.53 mg/mL) and stir for 1.5 hours at room temperature. Store conjugate at 20̸^{o}C.

### b) Detection of Human Autoantibodies to Pancreatic Islet Cells

25 uL of patient serum samples, diluted in 0̸.0̸5 M phosphate buffered saline (pH 7.4) [1:2, 1:8] are placed on tissue sections of slides layered with 4 micron thick sections of Monkey pancreas tissue. Incubate slides for 1 hr. in a humid chamber. Slides are then washed twice in PBS and incubated at 20̸^{o}C for 1 hour with streptavidin conjugate diluted in PBS applied onto the tissue section. After incubation, wash slides twice in PBS and add 1 mL of biotin labeled glucose oxidase (commercially available) diluted in PBS and incubate for an additional hour at 20̸^{o}C. Wash slides twice with PBS, blot and add 1.0̸ mL of 0̸.0̸0̸0̸5% nitroblue tetrazolium, .0̸0̸75% glucose and 0̸.0̸0̸0̸2% phenazinemethosulfate in 0̸.1 M NaH₂PO₄ buffer (pH 6.9). Incubate for 30̸ minutes at 20̸^{o}C, wash twice with PBS and permanently mount with suitable mounting media. The pancreas sections are evaluated using a light microscope. Positive staining is determined by observing cytoplasmic staining of the cells in the Islets of Langerhans. Each section is scored 0̸, 1+, 2+, 3+, 4+, etc. depending on the intensity of staining. Results are reported as titers.

### Example IV

A competitive immunoassay for the determination of human IgE in patient serum samples can be carried out in the following manner.

### a) Streptavidin is Conjugated to Affinity Purified Human IgE

This is done in the same manner described for the conjugation of human IgG to Streptavidin in Example III.

### b) Determination of Human IgE in Patient Serum Samples

To wells coated with a discrete quantity of rabbit antibody to human IgE add 10̸0̸ microliters of patient serum or standard diluted in PBS. Add 50̸ microliters of IgE-Streptavidin conjugate in PBS and incubate the mixture overnight (about 16 hours) at 20̸^{o}C. Aspirate and wash wells thoroughly with PBS prior to addition of 10̸0̸ microliters biotin labeled horseradish peroxidase. Incubate for 1 hour at 20̸^{o}C. Aspirate, wash as before and add 20̸0̸ microliters of substrate buffer containing 0̸.3% o-Phenylenediamine·2 HCl and 0̸.0̸2% Hydrogen Perosidase Citrate-Phosphate buffer (pH 5.2). Incubate for 30̸ minutes at 20̸^{o}C and stop reaction by addition of 50̸ uL 2 N H₂SO₄. IgE concentrations will be inversely proportional to absorbance values at 492 nm.

### Example V

Detection of adenovirus by DNA hybridization can be carried out in the following manner.

### a) Production of Two Distinct Adenovirus Derived Single Stranded DNA Fragments

This is described by Virtanen, M. et al., Lancet, Vol. I, p. 831 (1983).

### b) Conjugation of M13mp7 Phage Derived Single Stranded Adenovirus DNA Fragment to Streptavidin

30̸ mg Streptavidin is dissolved in 1.0̸ mL 0̸.0̸1 M phosphosaline buffer (PBS) (pH 7.3). While stirring add 10̸ microliters 0̸.1 M N-succinimidyl-S-acetylthioacetate (SATA), in N-N-dimethylformamide (DMF) and incubate at 20̸^{o}C for 20̸ minutes. Elute mixture through a Sephadex® G-25 column with 1 M Tris-HCl (pH 8.0̸) and concentrate protein peak fraction to approximately 1.0̸ mL.

Mix 1 mg DNA in 10̸0̸ microliters 2M NH₂NH₂/1 M NaHSO₃, (pH 6.3) and incubate at 37^{o}C for 75 minutes. Add 1.4 mL 0̸.1 M sodium phosphate buffer (pH 7.6) and dialyze twice against this buffer (1.0̸ liter) for 24 hours. Follow the dialysis against 0̸.0̸5 M NaCl for an additional 12 hours. Precipitate and wash derivatized DNA with excess ethanol and resolubilize in 10̸0̸ microliters 1 M Tris-HCl (pH 8.0̸).

Treat 12 mg of SATA derivatized streptavidin in 1.0̸ mL 1M Tris-HCl (pH 8.0̸) with 4.0̸ mg bromopyruvic acid and incubate for 15 minutes. Mix with 5 mg of the prepared polynucleotide and allow to stand at 20̸^{o}C for 1 hour. Elute Streptavidin labeled probe in a Sephadex® G-10̸0̸ column with 0̸.15 M NaCl with .0̸15 M sodium citrate (SSC).

### c) Sandwich Hybridization

Denature pBR322 plasmid DNA in 0̸.3 NaOH at 10̸0̸^{o}C for 5 minutes. Cool to 0̸^{o}C, and mix with an equal volume of 2 M ammonium acetate. Apply onto 3 mm nitrocellulose filters (150̸ ng/filter). Prior to hybridization, preincubate filters in 0̸.15 NaCl with 0̸.0̸15 M sodium citrate (SSC), 0̸.0̸2% ficoll and 0̸.25% SDS. Sample DNA is denatured by boiling in 7 mM NaOH for 5 minutes. After cooling and neutralization, dilute 1:4 in SSC with 0̸.0̸2% ficoll and streptavidin labeled probe. Each hybridization reaction contains the filter with immobilized DNA, and 40̸0̸ microliters of sample/probe mixture. Incubate at 37^{o}C for 16 hours. Wash filter thoroughly in SSC with 0̸.0̸2% ficoll and biotin labeled peroxidase at 20̸^{o}C for 2 hours. Wash filter thoroughly in SSC with 0̸.0̸2% ficoll and develop in 10̸ mL of 0̸.1 M Tris-HCl (pH 7.4) with 10̸ mM imidazole, 10̸ microliters of 30̸% H₂O₂ and3,3'-dianidisine in 2 mL ethanol. Rinse and store filter in Tris-HCl.

## Claims

1. A method of determining a ligand in a medium suspected of containing same, which comprises:
(a) providing an insoluble phase containing a specific binding substance for the ligand;
(b) incubating said insoluble phase with the following reagents,
(i) the medium suspected of containing the ligand;
(ii) a soluble reagent selected from the group consisting of
(a') a specific binding substance for the ligand which is covalently linked with avidin reagent;
(b') said reagent (a') complexed with biotin labeled enzyme;
(c') ligand which is linked to avidin; or
(d') reagent (c') complexed with biotin labeled enzyme; and
(iii) soluble biotin labeled enzyme when said reagent is (a') or (c');
(c) Separating unreacted reagents from said insoluble phase after incubation; and
(d) determining the enzyme activity of either said separated unreacted reagents or said separated insoluble phase to determine the ligand.

2. The method as defined in claim 1 wherein in step (b) said insoluble phase is incubated with said specific binding substance covalently linked with avidin reagent bound to biotin labeled enzyme.

3. The method as defined in claim 1 wherein in step (b) said insoluble phase is incubated with ligand which is linked to avidin bound to biotin labeled enzyme.

4. The method as defined in claims 1, 2, or 3 wherein step (b) comprises incubating said insoluble phase with reagents (i), (ii) and where appropriate reagent (iii) in the presence of one another.

5. The method as defined in claims 1, 2, or 3 wherein step (b) comprises incubating said insoluble phase with reagents (i), (ii) and where appropriate reagent (iii) in the order indicated.

6. The method as defined in claim 5 which further comprises in step (c) separating unreacted reagent from said insoluble phase after each incubation, and step (d) comprises determining the enzyme activity of either said insoluble phase or separated unreacted biotin labeled enzyme.

7. The method as defined in claims 1, 2, or 3 wherein said ligand is an antigen, an antibody, a hapten or another substance having a receptor in a living organism.

8. A method for determining a ligand in a medium suspected of containing the same, which comprises:
(a) providing an insoluble phase containing a specific binding substance for said ligand;
(b) incubating said insoluble phase with the following reagents;
(i) (A) liquid medium suspected of containing said ligand and (B) a known quantity of a soluble reagent selected from the group consisting of
(a') a specific binding substance for the ligand which is covalently linked with an avidin reagent;
(b') said reagent (a') complexed with biotin labeled enzyme;
(c') ligand which is linked to avidin; or
(d') reagent (c') complexed with biotin labeled enzyme; and
(ii) soluble biotin labeled enzyme when said reagent is (a') or (c');
(c) separating unreacted reagents from said insoluble phase after incubation, and
(d) determining the enzyme activity of either said insoluble phase or separated unreacted reagent whereby said activity is related to the amount of ligand in said liquid medium.

9. The method as defined in claim 8 where in step (b) said known quantity of (B) comprises reagent (b').

10. The method as defined in claim 7 where in step (b) said known quantity of (B) comprises reagent (d').

11. The method as defined in claim 8 where in step (b) said known quantity of (B) comprises reagent (d')

12. The method as defined in claim 8 wherein step (b) comprises incubating said insoluble phase with reagents (i) and, where appropriate, (ii), in the order indicated; step (c) comprises separating unreacted reagent from said insoluble phase after each incubation; and step (d) comprises determining the enzyme activity of either said insoluble phase or separated unreacted biotin labeled enzyme.

13. The method as defined in claims 8, 9 wherein said ligand is selected from the group consisting of antigen, antibody, a hapten, or another substance having a receptor in a living organism.

14. A reagent for determining a ligand which comprises
(a) a specific binding substance for the ligand
(b) an avidin labeling group covalently bonded to said specific binding substance by means of a coupling reagent reacted therewith; and
(c) a biotin labeled enzyme group coupled to said covalently bound avidin labeling group.

15. The reagent as defined in claim 14 wherein said coupling reagent is a compound of the formula R¹-S-S-A-Z, where R¹ is 2-pyridyl, 5-nitro-2-pyridyl or 4-pyridyl, A is a hydrocarbon residue having 1-10 carbon atoms, and Z is a group selected from 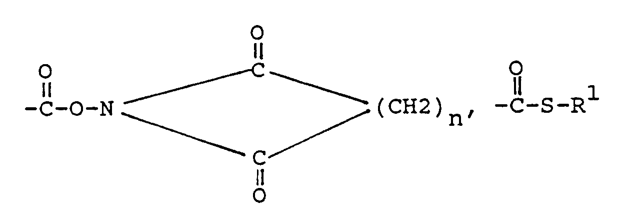 or 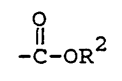 where n' is 2 or 3, R¹ has the same significance as R¹ above and is equal thereto, and R² is methyl or ethyl.

16. The reagent as defined in claim 14 wherein said coupling reagent is N-succinimidyl-3-(2-pyridylthio) propionate.

## Patentansprüche

1. Verfahren zur Bestimmung eines Liganden in einem Medium, in welchem der Ligand vermutlich enthalten ist, welches Verfahren umfaßt:
(a) Bereitstellen einer unlöslichen Phase, welche eine für den Liganden spezifische Bindungssubstanz enthält;
(b) Inkubieren dieser unlöslichen Phase mit den folgenden Reagentien:
(i) dem Medium, welches den Liganden vermutlich enthält;
(ii) einem löslichen Reagens, welches aus einer Gruppe ausgewählt ist, welche besteht aus:
(a') einer für den Liganden spezifischen Bindungssubstanz, welche mit einem Avidin-Reagens kovalent verknüpft ist;
(b') dem genannten Reagens (a'), welches mit biotinmarkiertem Enzym komplex gebunden ist;
(c') dem an Avidin gebundenen Liganden; oder
(d') einem Reagens (c'), welches mit biotinmarkiertem Enzym komplex gebunden ist; und
(iii) einem löslichen, biotinmarkiertem Enzym, wenn das genannte Reagens (a') oder (c') ist;
(c) Abtrennen der nicht-umgesetzten Reagentien von der unlöslichen Phase nach dem Inkubieren; und
(d) Bestimmung der Enzymaktivität von entweder den abgetrennten, nicht-umgesetzten Reagentien oder von der abgetrennten, unlöslichen Phase, um den Liganden zu bestimmen.

2. Verfahren nach Anspruch 1, wobei in Stufe (b) die unlösliche Phase mit der spezifischen Bindungssubstanz inkubiert wird, welche kovalent mit dem Avidin-Reagens verknüpft ist, welches seinerseits an das biotinmarkierte Enzym gebunden ist.

3. Verfahren nach Anspruch 1, wobei in Stufe (b) die unlösliche Phase mit dem Liganden inkubiert wird, welcher mit Avidin verknüpft ist, das seinerseits an biotinmarkiertes Enzym gebunden ist.

4. Verfahren nach den Ansprüchen 1, 2 oder 3, wobei die Stufe (b) das Inkubieren der unlöslichen Phase mit den Reagentien (i), (ii) und - sofern zutreffend - (iii) in Gegenwart aller dieser Reagentien umfaßt.

5. Verfahren nach den Ansprüchen 1, 2 oder 3, wobei die Stufe (b) das Inkubieren der unlöslichen Phase mit den Reagentien (i), (ii) und - sofern zutreffend - (iii) in der angegebenen Reihenfolge umfaßt.

6. Verfahren nach Anspruch 5, welches weiterhin in der Stufe (c) das Abtrennen des nicht-umgesetzten Reagens von der unlöslichen Phase nach jeder Inkubation umfaßt, und welches in der Stufe (d) die Bestimmung der Enzymaktivität von entweder der unlöslichen Phase oder von dem abgetrennten, nicht-umgesetzten, biotinmarkierten Enzym umfaßt.

7. Verfahren nach den Ansprüchen 1, 2 oder 3, wobei der Ligand ein Antigen, ein Antikörper, ein Hapten oder eine andere, einen Rezeptor aufweisende Substanz in einem lebenden Organismus ist.

8. Verfahren zur Bestimmung eines Liganden in einem Medium, in welchem der Ligand vermutlich enthalten ist, welches Verfahren umfaßt:
(a) Bereitstellen einer unlöslichen Phase, welche eine für den Liganden spezifische Bindungssubstanz enthält;
(b) Inkubieren dieser unlöslichen Phase mit den folgenden Reagentien:
(i) (A) einem flüssigen Medium, in welchem der Ligand vermutlich enthalten ist, und (B) mit einer bekannten Menge eines löslichen Reagens, welches aus einer Gruppe ausgewählt ist, welche besteht aus:
(a') einer für den Liganden spezifischen Bindungssubstanz, welche mit einem Avidin-Reagens kovalent verknüpft ist;
(b') dem genannten Reagens (a'), welches mit biotinmarkiertem Enzym komplex gebunden ist;
(c') dem an Avidin gebundenen Liganden; oder
(d') einem Reagens (c'), welches mit biotinmarkiertem Enzym komplex gebunden ist; und
(ii) einem löslichen, biotinmarkiertem Enzym, wenn das genannte Reagens (a') oder (c') ist;
(c) Abtrennen der nicht-umgesetzten Reagentien von der unlöslichen Phase nach dem Inkubieren; und
(d) Bestimmung der Enzymaktivität von entweder der unlöslichen Phase oder von dem abgetrennten, nicht-umgesetzten Reagens, wobei diese Aktivität mit der Menge des Liganden in dem flüssigen Medium in Beziehung steht.

9. Verfahren nach Anspruch 8, wobei in der Stufe (b) die bekannte Menge von (B) das Reagens (b') umfaßt.

10. Verfahren nach Anspruch 7, wobei in der Stufe (b) die bekannte Menge von (B) das Reagens (d') umfaßt.

11. Verfahren nach Anspruch 8, wobei in der Stufe (b) die bekannte Menge von (B) das Reagens (d') umfaßt.

12. Verfahren nach Anspruch 8, wobei die Stufe (b) das Inkubieren der unlöslichen Phase mit den Reagentien (i) und - soferne zutreffend - (ii) in der angegebenen Reihenfolge umfaßt; und wobei die Stufe (c) das Abtrennen von nicht-umgesetztem Reagens von der unlöslichen Phase nach jeder Inkubation umfaßt; und wobei die Stufe (d) die Bestimmung der Enzymaktivität von entweder der unlöslichen Phase oder von dem abgetrennten, nicht-umgesetzten, biotinmarkierten Enzym umfaßt.

13. Verfahren nach den Ansprüchen 8 und 9, wobei der Ligand aus einer Gruppe ausgewählt ist, welche aus Antigen, Antikörper, einem Hapten oder einer anderen, einen Rezeptor aufweisenden Substanz in einem lebenden Organismus besteht.

14. Reagens zur Bestimmung eines Liganden, welches enthält:
(a) eine für den Liganden spezifische Bindungssubstanz;
(b) eine Avidin-Markierungsgruppe, welche mit Hilfe eines damit umgesetzten Kupplungs-Reagens an diese spezifische Bindungssubstanz kovalent gebunden ist; und
(c) eine biotinmarkierte Enzymgruppe, welche an die genannte, kovalent gebundene Avidin-Markierungsgruppe gekuppelt ist.

15. Reagens nach Anspruch 14, wobei das Kupplungs-Reagens eine Verbindung der Formel R¹-S-S-A-Z ist, worin R¹ für 2-Pyridyl, 5-Nitro-2-pyridyl oder 4-Pyridyl steht, A einen Kohlenwasserstoffrest mit 1 bis 10 Kohlenstoffatomen darstellt und Z für eine aus 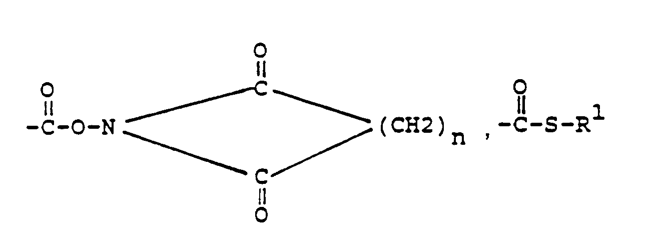 oder 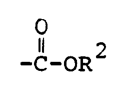 ausgewählte Gruppe steht, worin n für 2 oder 3 steht, R¹ die gleiche Bedeutung wie das obige R¹ hat und demselben gleich ist und R² für Methyl oder Ethyl steht.

16. Reagens nach Anspruch 14, wobei das Kupplungs-Reagens N-Succinimidyl-3-(2-pyridylthio)-propionat ist.

## Revendications

1. Procédé de détermination d'un ligand dans un milieu soupçonné le contenir, qui comprend:
(a) la fourniture d'une phase insoluble contenant une substance de liaison spécifique pour le ligand;
(b) l'incubation de ladite phase insoluble avec les réactifs suivants,
(I) le milieu soupçonné contenir le ligand;
(II) un réactif soluble choisi parmi
(a') une substance de liaison spécifique pour le ligand, qui est liée par covalence avec un réactif de type avidine;
(b') ledit réactif (a') complexé avec une enzyme marquée à la biotine;
(c') le ligand qui est lié à l'avidine; ou
(d') le réactif (c') complexé avec une enzyme marquée à la biotine; et
(III) une enzyme soluble marquée à la biotine, lorsque ledit réactif est (a') ou (c');
(c) la séparation des réactifs n'ayant pas réagi d'avec la phase insoluble après incubation; et
(d) la détermination de l'activité enzymatique soit desdits réactifs qui n'ont pas réagi et ont été séparés, soit de ladite phase insoluble séparée, pour déterminer le ligand.

2. Procédé selon la revendication 1, dans lequel, dans l'étape (b), on met ladite phase insoluble à incuber avec ladite substance de liaison spécifique liée par covalence à un réactif de type avidine fixé à une enzyme marquée à la biotine.

3. Procédé selon la revendication 1, dans lequel, dans l'étape (b), on met ladite phase insoluble à incuber avec un ligand qui est lié à de l'avidine fixée à une enzyme marquée à la biotine.

4. Procédé selon la revendication 1, 2 ou 3, dans lequel l'étape (b) comprend l'incubation de ladite phase insoluble avec les réactifs (I), (II) et, lorsque cela est approprié, le réactif (III) ensemble.

5. Procédé selon la revendication 1, 2 ou 3, dans lequel l'étape (b) comprend l'incubation de ladite phase insoluble avec les réactifs (I), (II) et, lorsque cela est approprié, le réactif (III), dans l'ordre indiqué.

6. Procédé selon la revendication 5, qui comprend en outre, dans l'étape (c), la séparation du réactif n'ayant pas réagi d'avec ladite phase insoluble, après chaque incubation, et l'étape (d) comprend la détermination de l'activité enzymatique soit de ladite phase insoluble, soit de l'enzyme marquée à la biotine qui n'a pas réagi et a été séparée.

7. Procédé selon la revendication 1, 2 ou 3, dans lequel ledit ligand est un antigène, un anticorps, un haptène ou une autre substance comportant un récepteur dans un organisme vivant.

8. Procédé pour la détermination d'un ligand dans un milieu soupçonné le contenir, qui comprend:
(a) la fourniture d'une phase insoluble contenant une substance de liaison spécifique pour ledit ligand;
(b) l'incubation de ladite phase insoluble avec les réactifs suivants,
(I) (A) un milieu soupçonné contenir ledit ligand et (B) une quantité connue d'un réactif soluble choisi parmi:
(a') une substance de liaison spécifique pour le ligand qui est liée par covalence avec un réactif de type avidine;
(b') ledit réactif (a') complexé avec une enzyme marquée à la biotine;
(c') un ligand qui est lié à l'avidine; ou
(d') un réactif (c') complexé avec une enzyme marquée à la biotine; et
(II) une enzyme soluble marquée à la biotine, lorsque ledit réactif est (a') ou (c');
(c) la séparation des réactifs n'ayant pas réagi d'avec ladite phase insoluble, après l'incubation; et
(d) la détermination de l'activité enzymatique soit de ladite phase insoluble, soit du réactif n'ayant pas réagi et séparé, ladite activité étant en relation avec la quantité de ligand dans ledit milieu liquide.

9. Procédé selon la revendication 8, dans lequel, dans l'étape (b), ladite quantité connue de (B) comprend le réactif (b').

10. Procédé selon la revendication 7, dans lequel, dans l'étape (b), ladite quantité connue de (B) comprend le réactif (d').

11. Procédé selon la revendication 8, dans lequel, dans l'étape (b), ladite quantité connue de (B) comprend le réactif (d').

12. Procédé selon la revendication 8, dans lequel l'étape (b) comprend l'incubation de ladite phase insoluble avec le réactif (I) et, lorsque cela est approprié, le réactif (II), dans l'ordre indiqué; l'étape (c) comprend la séparation du réactif n'ayant pas réagi d'avec ladite phase insoluble, après chaque incubation; et l'étape (d) comprend la détermination de l'activité enzymatique soit de ladite phase insoluble, soit de l'enzyme marquée à la biotine qui n'a pas réagi et a été séparée.

13. Procédé selon les revendications 8 et 9, dans lequel ledit ligand est choisi parmi un antigène, un anticorps, un haptène et une autre substance comportant un récepteur dans un organisme vivant.

14. Réactif pour la détermination d'un ligand, qui comprend
(a) une substance de liaison spécifique pour le ligand;
(b) un groupe marqueur de type avidine lié par covalence à ladite substance de liaison spécifique, au moyen d'un réactif de couplage ayant réagi avec celle-ci; et
(c) un groupe de type enzyme marqué à la biotine couplé audit groupe marqueur de type avidine lié par covalence.

15. Réactif selon la revendication 14, dans lequel ledit réactif de couplage est un composé de formule R¹-S-S-A-Z, dans laquelle R¹ est le groupe 2-pyridyle, 5-nitro-2-pyridyle ou 4-pyridyle, A est un radical hydrocarboné ayant de 1 à 10 atomes de carbone, et Z est un groupe choisi parmi 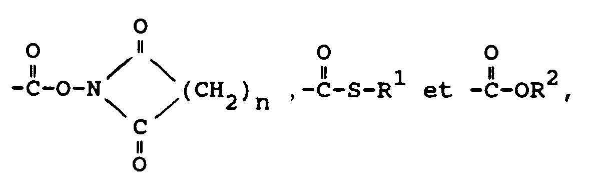 n étant 2 ou 3, R¹ ayant la même signification que R¹ ci-dessus et étant équivalent à celui-ci, et R² étant le groupe méthyle ou éthyle.

16. Réactif selon la revendication 14, dans lequel ledit réactif de couplage est le 3-(2-pyridylthio)propionate de N-succinimidyle.
